# EUROPÄISCHE PATENTSCHRIFT

(11) **EP 2 317 864 B1**
(45) Veröffentlichungstag und Bekanntmachung des Hinweises auf die Patenterteilung: **14.06.2017**
(21) Anmeldenummer: 09761348.3
(22) Anmeldetag: 08.04.2009
(51) Int. Cl.: A23K 20/00, A23L 33/10, A23C 3/08, A23L 2/44, A23L 3/3562, C12C 11/11, A23K 20/158, A23K 20/163, A23L 33/12, A23C 9/13, A61K 8/60, A61K 31/7016, C12C 5/02, C12C 12/02, A23L 27/30, A61Q 19/00

(54) **VERWENDUNG VON TREHALULOSE ALS HOCHWIRKSAMES ANTIOXIDATIONSMITTEL**
USE OF TREHALULOSE AS HIGHLY ACTIVE ANTIOXIDANT
UTILISATION DE TRÉHALULOSE COMME AGENT ANTIOXYDANT TRÈS EFFICACE

(30) Priorität: 11.06.2008 DE 102008027686; 25.11.2008 DE 102008060009
(43) Veröffentlichungstag der Anmeldung: 11.05.2011
(73) Patentinhaber: Südzucker AG Mannheim/Ochsenfurt, 68165 Mannheim (DE)
(72) Erfinder: KOWALCZYK, Jörg, 67304 Eisenberg/Steinborn (DE); HAUSMANNS, Stephan, 69126 Heidelberg (DE); DÖRR, Tillmann, 67591 Hohen-Sülzen (DE)
(74) Vertreter: Schwahn, Hartmut
(86) Internationale Anmeldenummer: PCT/EP2009/002599
(87) Internationale Veröffentlichungsnummer: WO 2009/149785

(56) Entgegenhaltungen:
- EP-A2- 0 483 755
- EP-A2- 0 794 259
- WO-A1-2005/061690
- WO-A2-2008/145247
- DE-A1-102005 034 043
- JP-A- 5 227 889
- US-A- 4 948 616
- US-A- 5 824 521
- US-A1- 2006 018 868
- OKU KAZUYUKI ET AL: "NMR and quantum chemical study on the OH...pi and CH...O interactions between trehalose and unsaturated fatty acids: implication for the mechanism of antioxidant function of trehalose" JOURNAL OF THE AMERICAN CHEMICAL SOCIETY, AMERICAN CHEMICAL SOCIETY, WASHINGTON, DC. US, Bd. 125, Nr. 42, 22. Oktober 2003 (2003-10-22), Seiten 12739-12748, XP002490210 ISSN: 0002-7863
- OKU KAZUYUKI; ET AL: "Combined NMR and quantum chemical studies on the interaction between trehalose and dienes relevant to the antioxidant function of trehalose" JOURNAL OF PHYSICAL CHEMISTRY PART B: CONDENSED MATTER, MATERIALS, SURFACES, INTERFACES & BIOPHYSICAL, AMERICAN CHEMICAL SOCIETY, Bd. 109, Nr. 7, 24. Februar 2005 (2005-02-24), Seiten 3032-3040, XP002490211 ISSN: 1520-6106 [gefunden am 2005-01-22]

## Beschreibung

Die Erfindung betrifft das technische Gebiet der Zusatz- oder Hilfsstoffe für Lebensmittel, Futtermittel, Kosmetika und Pharmazeutika besonders antioxidativ wirkende Zusatzstoffe und Antioxidantien. Die vorliegende Erfindung stellt die Verwendung von Trehalulose als Antioxidationsmittel für Lebensmittel, Futtermittel, Kosmetika und Pharmazeutika bereit, bevorzugt die Verwendung dieses Stoffes als einzigem hinzugegebenen antioxidativ wirkenden Zusatzstoff in Lebensmitteln, Futtermitteln, Kosmetika und Pharmazeutika. Antioxidativ wirkende Zusatz- oder Hilfsstoffe für Lebensmittel, pharmazeutische oder kosmetische Mittel sind bekannt. Sie unterdrücken vor allem das Auftreten von Abbauprodukten, die bei Herstellung oder Lagerung des Lebensmittels, Futtermittels, Kosmetikums oder Pharmazeutikums beim Kontakt oxidationsempfindlicher Inhaltsstoffe mit Luftsauerstoff oder anderen oxidativ wirkenden Substanzen entstehen. Im Folgenden wird auf Lebensmittel auf eine Weise Bezug genommen, dass darunter nicht nur bevorzugt Lebensmittel und Futtermittel verstanden werden, sondern auch alle weiteren im und am tierischen oder menschlichen Körper anwendbaren Mittel und Zusammensetzungen wie Kosmetika und Pharmazeutika. Als Lebensmittelzusatzstoffe wirken Antioxidantien zur Verbesserung der Lagerstabilität oder Alterungsstabilität der Lebensmittel. Sie wirken gegebenenfalls auch zusätzlich als therapeutisch und/oder prophylaktisch wirkender Wirkstoff, der seine Wirkung im tierischen oder menschlichen Körper entfaltet und dort schädliche oxidative Vorgänge unterdrückt. Ein Beispiel eines solchen Antioxidans ist Ascorbinsäure (Vitamin C). Ascorbinsäure und ihre Salze werden zum Beispiel Limonaden, Marmeladen, Kondensmilch oder Fleischprodukten beigefügt.

Antioxidationsmittel sind Lebensmittelzusatz- oder Hilfsstoffe, die in der Regel im Vergleich zu einem oxidationsempfindlichen Substrat in geringerer Konzentration vorliegen und dessen Oxidation deutlich verzögern oder verhindern. Neben der Eigenschaft, selbst ein Reduktionsmittel und damit ein Substrat oxidativer Vorgänge zu sein, können sich Antioxidationsmittel dadurch auszeichnen, dass sie Metallionen, beispielsweise zweiwertiges Eisen, welche eine katalytische Wirkung in Oxidationsreaktionen besitzen, in Form von Chelaten binden und/oder Radikal-Kettenreaktionen durch Abfangen des Starterradikals (Scavenging oder Quenchen) oder durch Abfangen eines Intermediärradikals (Chain-Breaking) unterbinden.

Viele Antioxidantien, darunter auch die Ascorbinsäure, haben auf Lebensmittel einen teilweise erheblich geschmacksveränderten Effekt. Dieser kann gezielt eingesetzt werden. So hat Ascorbinsäure einen säuerlichen, an Zitronensäure erinnernden Geschmack. In der Regel ist die antioxidative Wirkung des Antioxidationsmittels vom pH-Wert abhängig. So besitzt Ascorbinsäure hohe antioxidative Wirkung nur im Sauren. In vielen Lebensmitteln ist jedoch der säuerliche Geschmack unerwünscht oder es ist nicht möglich, ein saures Milieu einzuhalten, beispielsweise in Frischmilchprodukten. Inzwischen wurden für einige bekannte Antioxidationsmittel nachteilige gesundheitliche Auswirkungen auf den tierischen oder menschlichen Körper entdeckt. Darunter zählt auch die Ascorbinsäure sowie Schwefeldioxid und seine Salze (Sulfit, Bisulfit, Disulfit, Hydrogensulfit). Darüber hinaus wirken viele der bekannten Antioxidationsmittel zu stark reduzierend und führen deshalb unter bestimmten Bedingungen zu unerwünschten Reaktionen mit Bestandteilen des Lebensmittels.

Viele bekannte Antioxidationsmittel, so zum Beispiel die Ascorbinsäure, sind selbst sehr oxidations- und/oder lichtempfindlich, was ihre technologische Verarbeitbarkeit erschwert.

Aus der Publikation Oku et al., J. Phys. Chem. B 2005, 109 : 3032-3040 und aus Oku et al., J. Am. Chem. Soc. 2003, 125 : 12739-12748 sind die antioxidativen Eigenschaften von Trehalose und deren antioxidative Wirkung im Zusammenhang mit ungesättigten Fettsäuren bekannt. Daneben werden darin auch die antioxidativen Wirkungen von Saccharose, Maltose, Neotrehalose, Maltit und Sorbit beschrieben.

Es besteht daher der Bedarf, weitere Substanzen der Verwendung als Antioxidationsmittel zugänglich zu machen, die bei antioxidativer Wirkung die Nachteile bekannter Antioxidationsmittel nicht aufweisen.

Ein weiterer Aspekt mit dem die Erfindung zusammenhängt ist die Brauereitechnologie, vor allem die Herstellung von Bier mit hoher Lagerfähigkeit und Mitteln dazu. Bier ist als geschmacklich instabiles Lebensmittel bekannt, das einem natürlichen Alterungsvorgang unterworfen ist. Die Geschmacksstabilität stellt ein wichtiges Qualitätsmerkmal eines zur Lagerung vorgesehenen Bieres dar. Generell wird das Ziel verfolgt, den ursprünglichen Charakter des Bieres von der Abfüllung bis zum Verbrauch beizubehalten. Der Alterungsvorgang ist vor allem durch den oxidativen Abbau von Bierinhaltsstoffen und den dabei entstehenden sogenannten "Alterungskomponenten" charakterisiert. Diese verändern den Geschmack des Bieres nachteilig. Wesentlich für den oxidativen Abbau ist der Sauerstoffeintrag nach der Gärung und bei der Abfüllung. Der molekulare Luftsauerstoff bildet reaktive Sauerstoffformen, vor allem das Hydroxylradikal. Das Hydroxylradikal oxidiert vor allem die im Bier vorhandenen Komponenten Ethanol, freie Fettsäuren und Isohumulone zu Aldehyden und Ketonen; das Hydroxylradikal dient auch als Starterradikal für Reaktionen zu weiteren Radikalformen, woraus wiederum Aldehyde entstehen.

Bier enthält von Hause aus eine Reihe reduzierend wirkender Inhaltstoffe, die die Bildung solcher nachteiliger Oxidationsprodukte über einen gewissen Zeitraum verhindern. Diese sogenannte endogene antioxidative Aktivität oder das endogene antioxidative Potential (EAP) des Bieres verhilft dem Bier zu einer gewissen Lagerstabilität. Im Bier enthaltene antioxidativ wirkende Inhaltstoffe sind vor allem Schwefeldioxid, freie Phenole, Polyphenole oder Xanthohumole erreicht. Ist die antioxidative Kapazität dieser Inhaltstoffe erschöpft, so ist das Ende der Lagerfähigkeit des Biers erreicht. Bier hat deshalb von sich aus nur eine begrenzte Lagerfähigkeit.

Schwefeldioxid wird teilweise während der Hauptgärung durch die verwendeten Gärhefen gebildet. Die Schwefeldioxidbildung ist jedoch vom Gärverlauf und von dem verwendeten Hefestamm abhängig. Soll die endogene antioxidative Kapazität des Bieres durch Schwefeldioxid erhöht werden, muss ein spezieller Gärverlauf und eine spezielle Hefeauswahl getroffen werden; die Flexibilität bei der Ausübung der Braukunst bleibt dabei eingeschränkt.

Um die Anteile an solchen phenolischen Substanzen im Bier zu erhöhen, werden verschiedene technologische Maßnahmen bei der Bierherstellung angewendet. Solche Verfahren sind teilweise aufwändig und verändern das Brauergebnis; außerdem wird die Flexibilität bei der Ausübung der Braukunst eingeschränkt.

Ein bekanntermaßen zu Bier zusätzlich zugesetztes Antioxidationsmittel ist Schwefeldioxid. Dies wird im Allgemeinen solchen Bieren zugesetzt, die für eine längere Lagerung, beispielsweise für den Export nach Übersee, vorgesehen sind. Schwefeldioxid verändert den Geschmack des Bieres von vornherein nachteilig. Andere bekannte Oxidationsmittel wie Ascorbinsäure verändern den Geschmack des Bieres ebenfalls nachteilig.

Es ist daher auch Aufgabe der vorliegenden Erfindung, die Verwendung eines Mittels bereitzustellen, und die endogene antioxidative Kapazität (EAP) von Bier, Biermischgetränken und ähnlichen Brauereierzeugnissen zu erhöhen, ohne geschmackliche Nachteile und andere mit bekannten Antioxidationsmitteln verbundene Nachteile in Kauf nehmen zu müssen.

Das der Erfindung zugrundeliegende technische Problem wird gelöst durch die Verwendung von Trehalulose oder Trehalulose-haltigem Sirup als Antioxidationsmittel oder als Bestandteil einer Antioxidationsmittelzusammensetzung vorzugsweise für den Einsatz in Lebensmitteln, Futtermittel, Kosmetika und Pharmazeutika. Bevorzugt wird erfindungsgemäß das Anti-oxidationsmittel Trehalulose oder Trehalulose-haltiger Sirup als einzige antioxidativ wirkende Komponente verwendet. Weiter steht im Zusammenhang mit der Erfindung, dass Trehalulose oder Trehalulosehaltiger Sirup Bestandteil einer Antioxidationsmittelzusammensetzung ist, die neben Trehalulose mindestens eine weitere Komponente aufweist, die die antioxidative Wirkung von Trehalulose oder des Trehalulose-haltigen Sirups synergistisch unterstützt. Eine solche synergistische Wirkung steht bevorzugt im Zusammenhang mit der Komplexierung oder Chelatierung von oxidierend, katalysierend wirkenden Metallionen.

Die Erfinder fanden überraschend, dass der Zusatz von Trehalulose oder Trehalulose-haltigem Sirup die oxidative Stabilität von Lebensmitteln oder Futtermitteln deutlich verbessert. Trehalulose wirkt als Hilfsstoff zur effizienten Verbesserung der Lagerstabilität, Alterungsstabilität oder Oxidationsstabilität. Trehalulose verhindert oder vermindert effektiv das Auftreten von Oxidations-Abbauprodukten, sogenannten Alterungskomponenten, die, beispielsweise in Lebensmitteln wie Bier oder bierähnlichen Getränken, durch ihre geschmacksverschlechternde und/oder gesundheitsgefährdende Wirkung die Lagerfähigkeit begrenzen. Trehalulose wirkt protektiv für oxidationsempfindliche Nahrungsmittelkomponenten wie Farbstoffe, Geschmacksstoffe und pharmazeutische Wirkstoffe, ungesättigte Fettsäuren, darunter vor allem omega-3 und/oder omega-6 Fettsäuren und vergleichbare Fettsäuren. Ohne an die Theorie gebunden sein zu wollen, zeigt Trehalulose überraschend eine deutlich größere und damit auch wirkungsvoll einsetzbare antioxidative Wirkung im Vergleich zu anderen bekannten reduzierenden Zuckern wie Glucose.

Die Erfindung sieht also vor, Substanz Trehalulose als Antioxidationsmittel bevorzugt für Lebensmittel, Kosmetikprodukte und pharmazeutische Präparate einzusetzen. Bevorzugt wird Trehalulose als einziges dem Produkt zugegebenes Antioxidationsmittel eingesetzt.

Trehalulose, alpha-D-Glucopyranosyl-1,1-beta-D-fructofuranose ist ein Disaccharid aus Glucose und Fructose und ein Strukturisomer der Saccharose. Sie wird vor allem durch Isomerisierung aus Saccharose gewonnen. Trehalulose-haltiger Sirup, der auch als Trehalulose-Sirup bezeichnet wird, wird bevorzugt aus Saccharose in einem biokatalysierten Prozess unter Verwendung immobilisierter und nicht vermehrungsfähiger Zellen von *Pseudomonas mesoacidophila,* beispielsweise des Stamms MX-45, oder entsprechender anderer transgener Organismen, bevorzugt bei einer Temperatur von 10 bis 10°C in an sich bekannter Weise gewonnen. Der erhaltene Roh-Sirup enthält Trehalulose in einem Anteil von 75 bis 92 Gew.-%, häufiger von 85 bis 90 Gew.-% (Gewicht der Trockensubstanz). Dieser kann unmittelbar als Trehalulose-haltiger Sirup gemäß der Erfindung eingesetzt werden. Durch bekannte Verfahren, beispielsweise Chromatographie und Kristallisation kann der Anteil an Trehalulose erhöht und der Anteil and Fremdsubstanzen und Rest-Saccharose abgereichert werden.

Trehalulose-haltige Sirupe verwendet gemäß der Erfindung weisen typischerweise folgende Zusammensetzung auf:

| | Norm% (HPLC-NH2) | g/100g TS (GC) |
|---|---|---|
| Trehalulose | 89,3-89,5 | 88,9 |
| Fructose | 0,2 | 0,32 |
| Glucose | 0,2 - 0,3 | 0,38 |
| Saccharose | 0,3-0,4 | 0,17 |
| Isomaltulose | 6,8 | 11 |
| Isomaltose | 0,2 - 0,3 | 0 |
| DP-3 | <0,1 | |
| Isomelezitose | <0,1 | |
| Rest | 2,7 | 0,94 |

Es versteht sich, dass diese Zusammensetzung in gewissem Rahmen abweichen und schwanken kann. Trehalulose-Sirupe verwendet gemäß der Erfindung enthalten gegebenenfalls Leucrose in einem Anteil von etwa 1%. Trehalulose-Sirupe enthalten gegebenenfalls auch Rest-Saccharose in einem Anteil von etwa 1%.

Die Erfindung ist nicht auf diese Verwendung von Trehalulose-Sirupe beschränkt. Im Zusammenhang mit dieser Erfindung wird unter einem Trehalulose-Sirup eine Trehalulose-haltige Zusammensetzung verstanden, die zumindest 60 Gew.-% Trehalulose enthält, bevorzugt aber 70 Gew.-% oder mehr vorzugsweise 80 Gew.-% oder mehr.

In bevorzugten Ausführungen ist der Anteil an Isomaltulose geringer und beträgt stets weniger als 15% und gegebenenenfalls weniger als 5%. Isomaltulose kann das Auftreten von Kristallisation im Endprodukt bedingen; dies kann je nach Endprodukt unerwünscht sein. In anderen bevorzugten Ausführungen ist keine Isomaltulose im Trehalulose-Sirup enthalten. Solche Varianten können beispielsweise durch Kristallisation der Nebenprodukte, vor allem Isomaltulose, erhalten werden.

Trehalulose und Trehalulose-haltige Sirupe waren bisher primär als Ersatz von Saccharose in bekannten Saccharose-haltigen Lebensmittelformulierungen vorgesehen. Trehalulose würde dabei vor allem als körpergebendes Süßungsmittel ("bulk substance") verwendet werden. Die vorliegende Erfindung stellt hingegen eine andere Lehre bereit: Trehalulose oder Trehalulose-haltige Sirupe werden als antioxidativ wirkende Hilfsstoffe oder Zusatzstoffe (Antioxidationsmittel) vor allem für Lebensmittel und Futtermittel, aber auch für kosmetische und pharmazeutische Zusammensetzungen eingesetzt. Die Erfindung liegt somit in einem anderen technischen Anwendungsbereich. Gemäß der Erfindung kann Trehalulose auch in Zusammensetzungen und Lebensmitteln eingesetzt werden, in denen die süßende Wirkung und/oder die Funktion als körpergebende Komponente nicht erforderlich ist und/oder nicht zum Tragen kommt. Die erfindungsgemäße Verwendung von Trehalulose oder Trehalulose-haltiger Sirupe liegt also auch außerhalb des Bereichs der Süßwaren und/oder außerhalb der kohlenhydrathaltigen Lebensmittel. Dazu zählen beispielsweise eiweiß- und/oder fettreiche Lebensmittel wie Molkereiprodukte (Käse, Joghurt etc.) oder öl- oder fetthaltige Zubereitungen (Margarine, Speiseöle etc.) sowie Futtermittel mit hohem Anteil an oxidationsempfindlichen Fettsäuren, beispielsweise für die Milchproduktion.

Es versteht sich, dass die Gegenwart anderer antioxidativ wirkender Substanzen in einer erfindungsgemäßen Verwendung einer Trehalulose-Zusammensetzung die antioxidative bzw. protektive Wirkung von Trehalulose unterstützen können.

Der Erfinder fanden überraschend, dass die Gegenwart von Isomaltulose in einer Trehalulose-Zusammensetzung die antioxidative bzw. protektive Wirkung von Trehalulose überadditiv oder synergistisch verbessert. Eine besonders bevorzugte Ausführung der Erfindung ist die Verwendung einer Zusammensetzung, enthaltend Isomaltulose und Trehalulose als die, bevorzugt einzigen, antioxidativ bzw. protektiv wirkenden Komponenten in Lebensmitteln, Futtermitteln, Kosmetika oder Pharmazeutika. In einer Variante dieser Ausführung beträgt der Anteil an Trehalulose in dieser Zusammensetzung mindestens 50 Gew.-% oder mehr und der Anteil an Isomaltulose 50 Gew.-% oder weniger. In einer anderen Variante ist die Trehalulose diejenige Komponente, die die antioxidative bzw. protektive Wirkung von Isomaltulose überadditiv verbessert. In dieser Variante beträgt der Anteil an Trehalulose in dieser Zusammensetzung 50 Gew.-% oder weniger und der Anteil an Isomaltulose 50 Gew.-% oder mehr. Gegenstand der Erfindung ist demgemäß auch die Verwendung von Trehalulose oder Trehalulose-haltigem Sirup zur Verbesserung/Steigerung der antioxidative bzw. protektive Wirkung von Isomaltulose in Isomaltulose-haltigen Zusammensetzungen.

Die Erfindung steht damit im Zusammenhang mit Lebensmitteln, Kosmetikprodukten und pharmazeutischen Präparaten, welche das erfindungsgemäße Antioxidationsmittel enthalten. Die Erfindung betrifft auch die Verwendung von Trehalulose oder Trehalulose-haltigem Sirup als Antioxidationsmittel in einem solchen Produkt. Vorzugsweise enthält das Produkt Trehalulose oder Trehalulose-haltigen Sirup als einziges dem Produkt zugegebenes Antioxidationsmittel. Dabei ist nicht ausgeschlossen, dass zu der Trehalulose mindestens eine weitere Komponente beigefügt ist, die, vorzugsweise im Zusammenhang mit einer synergistischen Wirkungsweise, den antioxidativen Effekt von Trehalulose unterstützt oder steigert.

Ein Lebensmittel, welches Trehalulose oder Trehalulose-haltigen Sirup als Antioxidationsmittel enthält, ist bevorzugt ausgewählt aus:
i. Milcherzeugnissen und Milchprodukten wie Käse-, Butter-, Joghurt-, Kefir-, Quark-, Sauermilch-, Buttermilch-, Sahne-, Kondensmilch-, Trockenmilch-, Molken-, Milchzucker-, Milcheiweiß-, Milchmisch-, Milchhalbfett-, Molkenmisch- oder Milchfett-Produkte oder -Zubereitungen;
ii. Pudding, Creme, Mousse und andere Desserts;
iii. Milchfetterzeugnissen, Mischfetterzeugnissen, Speisefetten, Speiseölen;
iv. Backwaren wie Brot einschließlich Kleingebäck und feinen Backwaren, Dauerbackwaren, Keksprodukten und Waffeln;
v. Brotaufstrichen, insbesondere fetthaltigen Brotaufstrichen, Margarine-Erzeugnissen und Backfetten;
vi. Instantprodukten und Brüherzeugnissen;
vii. Obstprodukten oder -zubereitungen wie Konfitüren, Marmeladen, Gelees, Obstkonserven, Fruchtpülpe, Fruchtmark, Fruchtsäften, Fruchtsaftkonzentraten, Fruchtnektar und Fruchtpulver;
viii. Cerealien, Müsli und Cerealien-Mischungen, sowie fertig zubereiteten cerealien-haltigen Produkten wie Müsli-Riegel und Frühstücksprodukten;
ix. primär nicht-alkoholischen Getränken, Getränkegrundstoffen und Getränkepulvern, Kakaogetränken, Kakaogetränkepulvern;
x. primär alkoholischen Getränken und Gärprodukten, Wein, Weinmischgetränken, Bier, Biermischgetränken, alkoholfreiem Bier oder Biermischgetränk, alkoholreduziertem Bier oder Biermischgetränk;
xi. Fleischwaren und Wurstwaren;
xii. Süßwaren wie Schokoladen, Hartkaramellen, Weichkaramellen, Kaugummi, Dragees, Fondant-Erzeugnissen, Gelee-Erzeugnissen, Lakritzen, Schaumzuckerwaren, Flocken, Dragees, Komprimaten, kandierten Früchten, Krokant, Nougat-Erzeugnissen, Eiskonfekt, Marzipan, Speiseeis.

Selbstverständlich steht die Erfindung auch im Zusammenhang mit Lebensmitteln oder Futtermitteln, die von den vorgenannten Produkten abgeleitet sind, insbesondere mit diätetischer Spezialnahrung. Weiter mit Lebensmitteln, die nicht oder nicht ausschließlich für den menschlichen Verzehr gedacht oder geeignet sind; dazu zählen Futtermittel, Tiernahrung, Vormischungen für Tiernahrung, stärkereiche Futtermittel, eiweißreiche Futtermittel, fettreiche Futtermittel, Kraftfutter und Konzentratkraftfutter. Es hat sich überraschend gezeigt, dass aufgrund der protektiven/antioxidativen Wirkung von Trehalulose beziehungsweise Trehalulose-Sirup der ursprüngliche Gehalt an oxidationsempfindlichen Fettsäuren im Futtermittel, beispielsweise omega-3 Fettsäure in Grünfutter oder Grünfutterzubereitungen, oder der Gehalt an zugesetzten Fettsäuren konserviert werden kann, und zwar nicht nur bei der Lagerung des Futtermittels, sondern auch noch während der Verdauung/Verwertung des Futters im Tier. Oxidationsempfindliche Nahrungsmittelbestandteile oder Zusatzstoffe stehen so in größerer Menge dem Tier zur Verfügung.

Ziel der erfindungsgemäßen Anwendung von Trehalulose oder Trehalulose-haltigem Sirup als Antioxidationsmittel sind Lebensmittel, die mindestens eine Komponente enthalten, die oxidationsempfindlich ist und Alterungsprozessen unterworfen ist, was die Haltbarkeit der Lagerfähigkeit des Lebensmittels vermindern würde. Darunter sind erfindungsgemäß Substanzen oder Substanzgemische zu verstehen, die, besonders bei der Herstellung und/oder der Lagerung der Lebensmittel, einem oxidativen Abbau unterworfen sind. Dieser oxidative Abbau wird bevorzugt durch den Kontakt mit sauerstoffhaltigen Komponenten, vor allem durch Kontakt mit Luftsauerstoff ausgelöst. Der oxidative Abbau kann außerdem durch im Lebensmittel oder in einer Lebensmittelzusammensetzung enthaltenen weiteren Substanzen, die selbst oxidativ wirken, verursacht werden. Darunter zählen beispielsweise oxidierende Säuren, Metalle in hoher Oxidationsstufe und deren Verbindungen, oxidierend wirkende Konservierungsmittel sowie andere oxidierend wirkende Sauerstoff-, Schwefel-, oder Halogen-Verbindungen. Trehalulose oder Trehalulose-haltiger Sirup als Antioxidationsmittel steigert in solchen Produkten auch die Stabilität gegenüber freien Radikalen und unterdrückt die Bildung freier Radikale.

Die Erfindung steht im Zusammenhang mit einem Lebensmittel, welches Trehalulose oder Trehalulose-haltigen Sirup als, bevorzugt einziges, Antioxidationsmittel enthält und ein Milchprodukt oder Milchmischprodukt, vor allem ein Joghurt ist und als oxidationsempfindliche Komponente ungesättigte Fettsäure, vor allem omega-3 Fettsäure, omega-6 Fettsäure und/oder ähnliche enthält. Es hat sich überraschend gezeigt, dass Trehalulose als Antioxidationsmittel den oxidativen Abbau von omega-3 Fettsäure und omega-6 Fettsäure wirksam unterdrückt. In einem Milchprodukt, besonders einem Joghurt, welches Trehalulose enthält, wird zugesetzte omega-3 Fettsäure, omega-6 Fettsäure oder eine andere oxidationsempfindliche Komponente (siehe oben) auch bei längerer Lagerung nur in geringem Umfang abgebaut.

Weiter steht die Erfindung im Zusammenhang mit dem Lebensmittel Bier oder mit davon abgewandelten Formen wie Biermischgetränk oder alkoholfreiem oder alkoholreduziertes Bier oder Biermischgetränk, worin Trehalulose oder Trehalulose-haltiger Sirup als bevorzugt einziges, besonders bevorzugt einziges zugesetztes Antioxidationsmittel enthalten ist. Es hat sich überraschend gezeigt, dass ein Trehalulose-haltiges Bier eine besonders hohe Lagerstabilität aufweist. Auch bei langer Lagerung halten sich alterungsbedingte nachteilige Geschmacksveränderungen in tolerablen Grenzen. Trehalulose stabilisiert oxidationsempfindliche Komponenten des Biers und steigert den EAP-Wert Trehalulose verhindert die frühzeitige Anreicherung der Alterungskomponenten, die für den sogenannten "Alterungsgeschmack" von gelagertem Bier verantwortlich sind.

Gegenstand der Erfindung ist demgemäß auch die Verwendung von Trehalulose oder Trehalulose-haltigem Sirup zur Verbesserung der Alterungsstabilität Oxidationsstabilität und/oder der Lagerstabilität von Lebensmitteln, Futtermitteln, Kosmetika und Pharmazeutika besonders von oxidationsempfindlichen Lebensmitteln, Futtermitteln, Kosmetika und Pharmazeutika insbesondere von Bier, Biermischgetränken, Instantgetränken und Kakaoinstantgetränken; vor allem die Verwendung von Trehalulose oder Trehalulose-haltigem Sirup zur Verminderung des Auftretens geschmacksverschlechternder Alterungskomponenten in Bier oder Biermischgetränken. Ein Gegenstand der Erfindung ist schließlich auch die Verwendung von Trehalulose zur Verminderung der Oxidation von oxidationsempfindlichen Farbstoffen, Geschmacksstoffen, pharmazeutischen Wirkstoffen und/oder ungesättigten Fettsäuren, vor allem omega-3 Fettsäuren und ähnlichen in Lebensmitteln, insbesondere in Joghurt oder ähnlichen.

Die Erfindung wird anhand der Beispiele und Figuren näher illustriert, ohne dass diese beschränkend zu verstehen wären.

Die Figuren zeigen:
Figur 1: ESR-Messung (Signalintensität und EAP-Wert) an frisch hergestellten Ansätzen von Diätbier mit Zusatz von Trehalulose, Isomaltulose oder ohne Zusatz.
Figur 2: ESR-Messung (Signalintensität und EAP-Wert) der gealterten Ansätze (s.o.) nach 3 Monate Lagerung bei 20 °C im Dunkeln.

### Beispiel 1: Stabilisierung von omega-3 Fettsäuren in Milchprodukten

Es wurde die Fähigkeit von Trehalulose-haltigem Sirup (ca. 89 Gew.-% Trehalulose) untersucht, den oxidativen Abbau von omega-3 Fettsäuren, welche in einer Joghurtmatrix eingebettet sind, zu unterdrücken beziehungsweise zu vermindern.

Dazu wurde Vollfett-Joghurt (Joghurt, mild, 3,5 % Fett; Milram) eingesetzt, worin DHA CL (Lonza) als omega-3 Fettsäure eingerührt wurde. Die folgenden Joghurtzubereitungen (g pro 100 g) wurden hergestellt:

| | |
|---|---|
| Ansatz A (erfindungsgemäß): | 5 g Trehalulose-Sirup |
| Ansatz B: | 5 g Fructose |
| Ansatz C: | 5 g Saccharose |

In jeweils 240 g dieser Ansätze wurde jeweils 150 mg DHA-CL (Lonza) und etwa 40 mg einer gesättigten Fettsäure (C22:0) als interner Standard mittels Ultraturrax eingerührt. Alle Maßnahmen erfolgten unter Stickstoff-Schutzatmosphäre.

Unmittelbar nach der Herstellung der Joghurtzubereitungen wurden jeweils Nullproben gezogen und die Wiederfindungsrate der omega-3 Fettsäure in den frisch hergestellten Zubereitungen bestimmt.

Zur Bestimmung der omega-3 Fettsäuren wurden jeweils 0,85 ml der Joghurtzubereitung in ein Probenröhrchen (Eppendorf) pipetiert, gewogen und mit jeweils 1 ml tert-Butyl-methylether versetzt und intensiv geschüttelt. Nach etwa 3 min Schütteln wurden die Probenröhrchen für 3 min bei 13.000 Upm zentrifugiert. Anschließend wurden jeweils 200 µl des klaren Überstands entnommen und mit jeweils 100 µl THMS versetzt. Jeweils 1 µl dieser Lösung wurde in einen Gaschromatographen (Agilent, Typ 6890) injiziert, der in an sich bekannter Weise auf die Detektion der eingesetzten Fettsäuren optimiert war.

In allen Nullprobenansätzen wurden unterschiedslos Wiederfindungsraten von 96 bis 99 % der ursprünglich eingesetzten omega-3 Fettsäure gefunden.

Die hergestellten Joghurtzubereitungen wurden nun für 28 Tage bei 7°C gelagert. Die Proben wurden jeweils gaschromatographisch analysiert.

Ergebnis: Die Trehalulose-haltigen Zubereitungen (Ansatz A) die zeigen die höchsten Wiederfindungsraten bei den oxidationsempfindlichen Fettsäuren. Der Geschmack dieser Zubereitungen ist einwandfrei. Die mit Fructose beziehungsweise Saccharose versetzten Proben zeigten deutlich geringere Wiederfindungsraten. Geschmackliche Beeinträchtigungen treten auf (Fettgeschmack).

Überraschenderweise zeigt sich, dass Fructose, die über die Keto-En(di)ol-Tautomerie als reduzierender Zucker wirkt, selbst bei höherer Molarität einen deutlich geringeren protektiven Effekt als Trehalulose hat. Ohne an die Theorie gebunden sein zu wollen, ist die überraschend hohe antioxidative Wirkung von Trehalulose nicht allein auf die Gegenwart reaktiver Aldehydgruppen zurückzuführen. Die reduzierende Aktivität (Redox-Potential) von Aldehyd-Zuckern wurde allgemein als zu gering angesehen, als dass sich die gefundene hohe antioxidative Wirkung daraus allein herleiten könnte. Andere Mechanismen scheinen hier den antioxidativen und protektiven Effekt zu Unterstützen.

### Beispiel 2: Stabilisierung von Bier

Zur Überprüfung der Beeinflussung der Oxidationsstabilität von Bier wurde kommerzielles Diätbier (nahezu keine Kohlenhydrate enthaltend) mit Trehalulose versetzt (erfindungsgemäß). In Vergleichsproben wurde kommerzielles Diätbier mit Isomaltulose (Vergleich) versetzt oder das Diätbier ohne Zusatz verwendet. Alle Proben wurden einer definierten Bieralterung unterzogen. Die Messung der Oxidationsstabilität mittels ESR erfolgte am Beginn und am Ende der Bieralterung.

### kommerzielles Diätbier:

| | |
|---|---|
| Stammwürze [gem%] | 9,1 |
| Restextrakt scheinbar [Gew.-%] | 0 |
| Restextrakt wirklich [Gew.-%] | 1,66 |
| Alkoholgehalt [Vol.-%] | 4,78 |
| Bittereinheiten [BE] | 24 |

Ansatz 1: Diätbier mit 2 g/100 ml Trehalulose-Sirup (erfindungsgemäß)
Ansatz 2: Diätbier mit 1 g/100 ml Isomaltulose (Vergleichsbeispiel)
Ansatz 3: Diätbier mit 2 g/100 ml Isomaltulose (Vergleichsbeispiel)
Ansatz 4: Diätbier ohne Zusatz (Vergleichsbeispiel)

Alle Ansätze wurden 3 Monate bei 20 °C im Dunkeln gelagert (Bieralterungstest).

Die oxidative Stabilität der Probe wurde mittels ESR (Elektronenspinresonanzspektroskopie) nach der Methodik von Methner und Kunz (Methner und Kunz, 2006, Genauere Prognosen zur oxidativen Bierstabilität mittels ESR-Spektroskopie, Brauerei Forum 2006: 7 - 9) bestimmt. Dabei zeigt ein hoher EAP-Wert eine günstigere oxidative Stabilität an. Weiterhin wird die absolute Signalintensität zur Beurteilung herangezogen, da diese ein Maß für die während der Messung durch oxidative Vorgänge gebildeten radikalischen Substanzen ist.

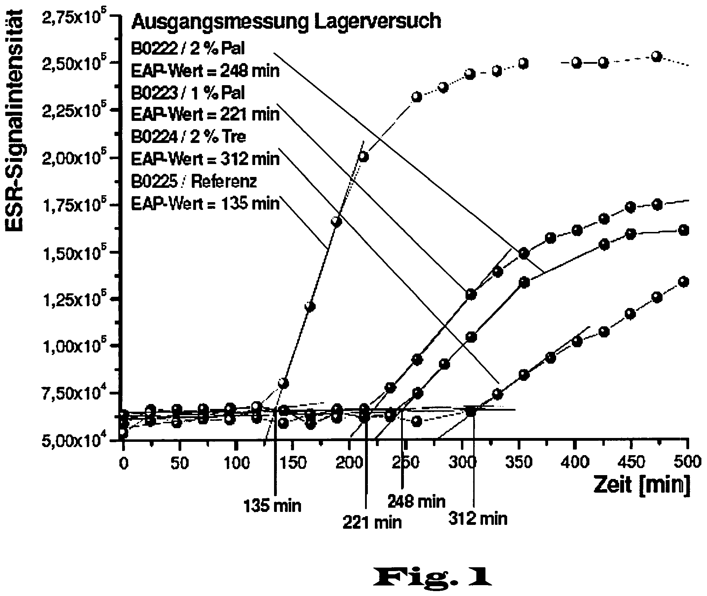

Figur 1 zeigt die Ergebnisse der ESR-Messung (Signalintensität und EAP-Wert) an den frisch hergestellten Ansätzen (s.o.) nach Kaltlagerung (0 bis 2 °C).

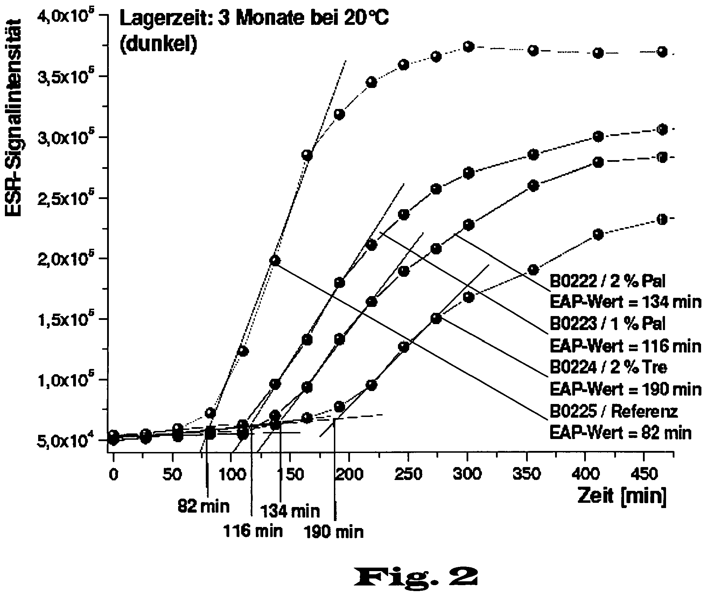

Figur 2 zeigt die Ergebnisse der ESR-Messung (Signalintensität und EAP-Wert) der gealterten Ansätze (s.o.) nach 3 Monate Lagerung bei 20 °C im Dunkeln.

Der EAP-Wert für den efindungsgemäßen Ansatz 1 mit Trehalulose ("2% Tre") ist am höchsten: EAP = 312 min im frischen Ansatz und EAP = 190 min im 3 Monate gelagerten Ansatz. Die ESR-Signalintensität ist im erfindungsgemäßen Ansatz 1 ("2% Tre") am geringsten. Dies weist auf das hohe antioxidative Potential von Trehalulose als antioxidativ wirkendes Agens hin.

Im Vergleich zeigt Isomaltulose (Ansätze 2 und 3; "1 % Pal" und "2% Pal") zwar ebenfalls eine antioxidative Wirkung. Diese ist aber bei gleicher zugesetzter Menge weniger stark ausgeprägt. Die Erfindung stellt mit der Verwendung von Trehalulose als antioxidativ wirkendem Agens ein Antioxidationsmittel bereit, das überraschenderweise weit stärker antioxidativ wirkt als andere Saccharide und insbesondere als andere niedrig glykämische Saccharide, vor allem wie das Disaccharid Isomaltulose.

## Patentansprüche

1. Verwendung von Trehalulose oder Trehalulose-haltigem Sirup als Antioxidationsmittel.

2. Verwendung von Trehalulose oder Trehalulose-haltigem Sirup nach Anspruch 1 in einem Lebensmittel, Futtermittel, Kosmetikum oder Pharmazeutikum.

3. Verwendung von Trehalulose oder Trehalulose-haltigem Sirup nach Anspruch 1 oder 2 als einziger dem Lebensmittel hinzugegebener antioxidativ wirkender Hilfsstoff.

4. Verwendung von Trehalulose oder Trehalulose-haltigem Sirup nach Anspruch 2 oder 3, wobei das Lebensmittel, Futtermittel, Kosmetikum oder Pharmazeutikum mindestens eine oxidationsempfindliche Komponente enthält, ausgewählt aus ungesättigten Fettsäuren.

5. Verwendung von Trehalulose oder Trehalulose-haltigem Sirup nach Anspruch 4, wobei die ungesättigte Fettsäure ausgewählt ist aus omega-3 Fettsäuren und omega-6 Fettsäuren.

6. Verwendung von Trehalulose oder Trehalulose-haltigem Sirup nach einem der Ansprüche 2 bis 5, wobei das Lebensmittel ausgewählt ist aus:
i. Milcherzeugnissen und Milchprodukten wie Käse-, Butter-, Joghurt-, Kefir-, Quark-, Sauermilch-, Buttermilch-, Sahne-, Kondensmilch-, Trockenmilch-, Molken-, Milchzucker-, Milcheiweiß-, Milchmisch-, Milchhalbfett-, Molkenmisch- oder Milchfett-Produkte oder - Zubereitungen;
ii. Pudding, Creme, Mousse und andere Desserts;
iii. Milchfetterzeugnissen, Mischfetterzeugnissen, Speisefetten, Speiseölen;
iv. Backwaren wie Brot einschließlich Kleingebäck und feinen Backwaren, Dauerbackwaren, Keksprodukten und Waffeln;
v. Brotaufstrichen, insbesondere fetthaltigen Brotaufstrichen, Margarine-Erzeugnissen und Backfetten;
vi. Instantprodukten und Brüherzeugnissen;
vii. Obstprodukten oder -zubereitungen wie Konfitüren, Marmeladen, Gelees, Obstkonserven, Fruchtpulpe, Fruchtmark, Fruchtsäften, Fruchtsaftkonzentraten, Fruchtnektar und Fruchtpulver;
viii. Cerealien, Müsli und Cerealien-Mischungen, sowie fertig zubereiteten cerealienhaltigen Produkten wie Müsli-Riegel und Frühstücksprodukten;
ix. primär nicht-alkoholischen Getränken, Getränkegrundstoffen und Getränkepulvern, Kakaogetränken, Kakaogetränkepulvern;
x. primär alkoholischen Getränken und Gärprodukten, Wein, Weinmischgetränken, Bier, Biermischgetränken, alkoholfreiem Bier oder Biermischgetränk, alkoholreduziertem Bier oder Biermischgetränk;
xi. Fleischwaren und Wurstwaren;
xii. Süßwaren wie Schokoladen, Hartkaramellen, Weichkaramellen, Kaugummi, Dragees, Fondant-Erzeugnissen, Gelee-Erzeugnissen, Lakritzen, Schaumzuckerwaren, Flocken, Dragees, Komprimaten, kandierten Früchten, Krokant, Nougat-Erzeugnissen, Eiskonfekt, Marzipan, Speiseeis;
sowie aus daraus abgeleiteter diätetischer Spezialnahrung.

7. Verwendung von Trehalulose oder Trehalulose-haltigem Sirup nach einem der Ansprüche 2 bis 5, wobei das Futtermittel Tiernahrung, Vormischung für Tiernahrung, stärkereiches, eiweißreiches oder fettreiches Futtermittel, Konzentratfutter oder Kraftfutter ist.

8. Verwendung von Trehalulose oder Trehalulose-haltigem Sirup nach Anspruch 6, wobei das Lebensmittel ein omega-3 oder omega-6 Fettsäure-haltiges Milchprodukt, Joghurt oder Milchmischprodukt ist.

9. Verwendung von Trehalulose oder Trehalulose-haltigem Sirup nach einem der Ansprüche 2 bis 5, wobei das Lebensmittel ein Bier, Biermischgetränk oder alkoholfreies oder alkoholreduziertes Bier oder Biermischgetränk ist.

10. Verwendung von Trehalulose oder Trehalulose-haltigem Sirup nach einem der Ansprüche 1 bis 9 zur Verbesserung der Oxidationsstabilität von Lebensmitteln, Futtermitteln, Kosmetika und Pharmazeutika.

11. Verwendung von Trehalulose oder Trehalulose-haltigem Sirup nach einem der Ansprüche 1 bis 9 zur Verminderung des Auftretens geschmacksverschlechternder Alterungskomponenten in Lebensmitteln, Futtermitteln, Kosmetika und Pharmazeutika.

12. Verwendung von Trehalulose oder Trehalulose-haltigem Sirup nach einem der Ansprüche 1 bis 9 zur Verminderung der Oxidation von ungesättigten Fettsäuren in Lebensmitteln, Futtermitteln, Kosmetika und Pharmazeutika.

## Claims

1. Use of trehalulose or trehalulose-containing syrup as antioxidant.

2. Use of trehalulose or trehalulose-containing syrup according to claim 1 in a food, animal feed, cosmetic or pharmaceutical.

3. Use of trehalulose or trehalulose-containing syrup according to claim 1 or 2 as the only adjuvant added to the food with an antioxidative effect.

4. Use of trehalulose or trehalulose-containing syrup according to claim 2 or 3, wherein the food, animal feed, cosmetic or pharmaceutical contains at least one oxidation-sensitive component selected from unsaturated fatty acids.

5. Use of trehalulose or trehalulose-containing syrup according to claim 4, wherein the unsaturated fatty acid is selected from omega-3 fatty acids and omega-6 fatty acids.

6. Use of trehalulose or trehalulose-containing syrup according to one of claims 2 to 5, wherein the food is selected from:
i. milk products and dairy products such as cheese, butter, yogurt, kefir, fresh cheese, sour milk, buttermilk, cream, condensed milk, powdered milk, whey, lactose, milk protein, mixed milk, low-fat milk, mixed-whey or butterfat products or preparations;
ii. pudding, crème, mousse and other desserts;
iii. butterfat products, mixed fat products, edible fats and edible oils;
iv. baked goods such as bread, including pastries and specialty baked goods, long-life cookies and cakes, biscuit products and wafers;
v. bread spreads, in particular fat-containing bread spreads, margarine products and shortenings;
vi. instant products and steeped beverages;
vii. fruit products or preparations such as preserves, marmalades, jellies, fruit compote, fruit pulps, fruit concentrate, fruit juices, fruit-juice concentrates, fruit nectar and powdered fruit juice;
viii. cereals, muesli and cereal mixtures as well as finished cereal-containing products such as muesli bars and breakfast products;
ix. primary non-alcoholic beverages, beverage bases and beverage powders, chocolate drinks, chocolate-drink powders;
x. primary alcoholic drinks and fermented products, wine, mixed wine beverages, beer, mixed beer beverages, alcohol-free beer or mixed beer beverage, reduced-alcohol beer or mixed beer beverage;
xi. meat products and sausage products;
xii. sweets such as chocolates, hard caramels, soft caramels, chewing gum, pellets, fondant products, jelly products, liquorices; foamed sweets, flakes, drops, compressed sweets, candied fruits, pralines, nougat products, ice-cream confections, marzipan and ice cream;
as well as dietary nutritional products derived therefrom.

7. Use of trehalulose or trehalulose-containing syrup according to one of claims 2 to 5, wherein the feed is an animal food, pre-mix for animal food, high starch, high-protein or high-fat feed, concentrated or high-energy feed.

8. Use of trehalulose or trehalulose-containing syrup according to one of claim 6, wherein the food is a milk product, yogurt or mixed milk product containing omega-3 or omega-6 fatty acid.

9. Use of trehalulose or trehalulose-containing syrup according to one of claims 2 to 5, wherein the food is a beer, mixed beer beverage or alcohol-free or reduced-alcohol beer or mixed beer beverage.

10. Use of trehalulose or trehalulose-containing syrup according to one of claims 1 to 9 for improving the oxidation stability of food, feed, cosmetics and pharmaceuticals.

11. Use of trehalulose or trehalulose-containing syrup according to one of claims 1 to 9 for reducing the appearance of aging components that negatively affect taste in food, feed, cosmetics and pharmaceuticals.

12. Use of trehalulose or trehalulose-containing syrup according to one of claims 1 to 9 for reducing the oxidation of unsaturated fatty acids in foods, feeds, cosmetics and pharmaceuticals.

## Revendications

1. Utilisation du tréhalulose, ou d'un sirop contentant du tréhalulose, comme agent antioxydant.

2. Utilisation du tréhalulose ou d'un sirop contentant du tréhalulose selon la revendication 1 dans un aliment, fourrage, produit cosmétique ou produit pharmaceutique.

3. Utilisation du tréhalulose ou d'un sirop contentant du tréhalulose selon la revendication 1 ou 2 comme seule agent auxiliaire avec effet antioxydant ajouté à l'aliment.

4. Utilisation du tréhalulose ou d'un sirop contentant du tréhalulose selon la revendication 2 ou 3, dans laquelle l'aliment, le fourrage, le produit cosmétique ou le produit pharmaceutique contient au moins un composant sensible à l'oxydation, le composant étant choisi parmi les acides gras non-saturés.

5. Utilisation du tréhalulose ou d'un sirop contentant du tréhalulose selon la revendication 4, dans laquelle l'acide gras non-saturé est choisi parmi les acides gras oméga 3 et les acides gras oméga 6.

6. Utilisation du tréhalulose ou d'un sirop contentant du tréhalulose selon l'une quelconque des revendications 2 à 5, dans laquelle l'aliment est choisi parmi :
i. produits laitiers comme produits et préparations de fromage, beurre, yaourt, kéfir, fromage blanc, lait fermenté, babeurre, crème, lait concentré, lait en poudre, petit-lait, sucre de lait, protéine de lait, produits et préparations mélangés à base de lait, de lait mi-gras, produits et préparations mixtes à base de petit-lait ou graisse du lait ;
ii. pouding, crème dessert, mousse et autres desserts ;
iii. produits de graisse de lait, de graisse mélangée, graisses et huiles comestibles ;
iv. produits de boulangerie comme pain, y compris les viennoiseries et les pâtisseries, biscottes, produits de biscuiterie et gaufres ;
v. pâtes à tartiner, notamment des pâtes à tartiner contenant de la matière graisse, produits de margarine et graisses pour boulangeries ;
vi. produits instantanés et produits à infuser ;
vii. produits et préparations de fruits, comme confitures, marmelades, gelées, fruits en conserve, pulpe de fruits, purée de fruits, jus de fruits, concentrés de jus de fruits, nectars de fruits et poudre de fruits ;
viii. céréales, muesli et mélanges de céréales, aussi que produits finis contenant des céréales, comme barres de céréales et produits pour petit déjeuner ;
ix. boissons et préparations de base de boissons et poudres de boisson essentiellement non alcoolisées, boissons cacaotées, poudres de boisson cacaotée ;
x. boissons essentiellement alcoolisées et produits fermentés, boissons mélangées à base de vin, bière, boissons mélangées à base de la bière, bière ou boissons à base de la bière sans alcool, bière ou boissons à base de la bière réduite en alcool ;
xi. produits de viande et charcuteries ;
xii. confiserie tels que chocolats, bonbons durs, bonbons mous, gommes à mâcher, dragées, produits fondants, produits de gelée, réglisse, produits de guimauve, flocons, dragées, produits comprimés, fruits confits, croquants/cassants, produits de nougat, bonbons de glace, massepain, crème glacée ;
et des aliments spéciaux diétiques dérivés de ceux-ci.

7. Utilisation du tréhalulose ou d'un sirop contentant du tréhalulose selon l'une quelconque des revendications 2 à 5, dans laquelle le fourrage est une alimentation animale, un prémélange d'une alimentation animale, un fourrage riche en amidon, riche en protéine ou riche en matière grasse, un fourrage concentré ou un aliment concentré.

8. Utilisation du tréhalulose ou d'un sirop contentant du tréhalulose selon la revendication 6, dans laquelle l'aliment est un produit laitière, un yaourt ou un produit mélangé à base de lait contenant de l'acide gras omega-3 ou omega-6.

9. Utilisation du tréhalulose ou d'un sirop contentant du tréhalulose selon l'une quelconque des revendications 2 à 5, dans laquelle l'aliment est une bière, une boisson mélangée à base de la bière ou une bière ou boisson à base de la bière sans alcool ou réduite en alcool.

10. Utilisation du tréhalulose ou d'un sirop contentant du tréhalulose selon l'une quelconque des revendications 1 à 9 pour améliorer la stabilité à l'oxydation des aliments, fourrages, produits cosmétiques ou produits pharmaceutiques.

11. Utilisation du tréhalulose ou d'un sirop contentant du tréhalulose selon l'une quelconque des revendications 1 à 9 pour réduire des composants de vieillissement effectuant la dégradation de la saveur dans les aliments, fourrages, produits cosmétiques ou produits pharmaceutiques.

12. Utilisation du tréhalulose ou d'un sirop contentant du tréhalulose selon l'une quelconque des revendications 1 à 9 pour réduire l'oxydation des acides gras non-saturés dans les aliments, fourrages, produits cosmétiques ou produits pharmaceutiques.
